# EUROPÄISCHE PATENTANMELDUNG

(11) **EP 0 654 275 A1**
(43) Veröffentlichungstag der Anmeldung: **24.05.1995**
(21) Anmeldenummer: 94114811.6
(22) Anmeldetag: 20.09.1994
(51) Int. Cl.: A61M 1/00, A61B 17/39

(54) **Chirurgische laparoskopische Vorrichtung**

(30) Priorität: 24.11.1993 DE 4340056
(71) Anmelder: DELMA ELEKTRO-UND MEDIZINISCHE APPARATEBAU GESELLSCHAFT mbH, D-78532 Tuttlingen (DE)
(72) Erfinder: Lurz, Michael, Dipl.-Ing. (FH), D-78589 Dürbheim (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(57) **Zusammenfassung**

Eine chirurgische laparoskopische Vorrichtung weist ein Schutzrohr (11) und einen an dessen Hinterseite angebrachten Handgriff (12) auf. In einem im Schutzrohr (11) und Handgriff (12) vorgesehenen axialen Führungskanal (14) ist wenigstens ein stabförmiges Instrument (13) axial herausnehmbar angeordnet. Parallel dazu verläuft im Schutzrohr und teilweise auch im Handgriff (12) ein Spül- oder Saugkanal (15), welcher durch den Handgriff (12) hindurch zu einem äußeren Spül- bzw. Sauganschluß (16, 17) geführt ist. Im Handgriff ist neben dem Führungskanal (14) ein Zweistellungs-Dreiwegeventil (19) angeordnet, welches in der einen Stellung den Spül- oder Saugkanal (15) mit dem Spül- bzw. Sauganschluß (16, 17) und in der anderen Stellung den Führungskanal (14) mit dem Spül- bzw. Sauganschluß (16 bzw. 17) verbindet.

## Beschreibung

Die Erfindung betrifft eine chirurgische laparoskopische Vorrichtung, insbesondere Behandlungsvorrichtung wie eine Multifunktionsbehandlungsvorrichtung nach dem Oberbegriff des Patentanspruchs 1. Die Vorrichtung ist insbesondere als Handgerät ausgebildet.

Derartige, zum Beispiel aus der DE-OS 42 42 143 bekannte Multifunktionsbehandlungsvorrichtungen weisen in Axialrichtung zumindest zwei Kanäle auf, in denen ein herausnehmbares Arbeitswerkzeug und ein Spül- oder Saugkanal angeordnet ist. In den Werkzeugführungskanal kann zum Beispiel ein Hochfrequenzzuleitungen aufnehmendes Rohr eingeschoben werden, welches an seinem distalen Ende beispielsweise zwei zangenartig zusammenführbare Koagulationselektroden trägt. Der Spül- oder Saugkanal kann über den Spülflüssigkeitszuführ- oder -absauganschluß mit einer Spülflüssigkeitszufuhr oder einer Absaugpumpe verbunden werden, so daß wahlweise Spülflüssigkeit durch den Kanal hindurchgeführt oder Wundflüssigkeit durch den Kanal abgesaugt werden kann.

Angesichts der Tatsache, daß das Schutzrohr derartiger Vorrichtungen einen möglichst geringen Querschnitt aufweisen soll, um zum Beispiel durch ein Trokar hindurch ungehindert an ein Operationsfeld zu gelangen, andererseits aber der Führungskanal angesichts des vorgegebenen Querschnittes des stabförmigen Instrumentes einen gewissen Mindestquerschnitt aufweisen muß, bleibt im allgemeinen für den Spül- oder Saugkanal im Schutzrohr und im Handgriff nur wenig Platz, so daß er einen vergleichsweise geringen Querschnitt aufweist. Hierdurch ist die Zufuhrmenge von Spülflüssigkeit oder auch die Absaugmenge von Sekret aus dem Operationsfeld begrenzt. Als besonders nachteilig erweist es sich, daß durch den geringen Querschnitt des Kanals im Schutzrohr leicht Verstopfungen durch aus dem Operationsfeld abgesaugtes stückiges Material vorkommen können.

Das Ziel der Erfindung besteht darin, eine weitere chirurgische laparoskopische Vorrichtung der eingangs genannten Gattung zu schaffen. Insbesondere soll es mit dieser Vorrichtung ohne eine Querschnittsvergrößerung des Schutzrohrs möglich sein, mehr Spülflüssigkeit oder auch abgesaugte Flüssigkeit zu fördern sind Verstopfungen wirksam zu vermeiden.

Zur Lösung dieser Aufgabe sind die Merkmale des kennzeichnenden Teils des Patentanspruchs 1 vorgesehen.

Vorteilhafte Weiterbildungen der Erfindung sind durch die Unteransprüche gekennzeichnet.

Der Grundgedanke der Erfindung ist darin zu sehen, daß der im allgemeinen einen deutlich größeren Querschnitt als der Spül- oder Saugkanal aufweisende Führungskanal zumindest im Bereich des Schutzrohres zusätzlich als Spülflüssigkeitszufuhr- bzw. -abfuhrkanal verfügbar gemacht wird, indem er bei herausgenommenem Instrument durch ein vorzugsweise selbsttätig schaltendes Ventil mit dem Spül- bzw. Sauganschluß verbunden wird. Auf diese Weise steht der sich durch das Schutzrohr und zumindest teilweise auch durch den Handgriff erstreckende Führungskanal auch für Spül- oder Absaugzwecke zur Verfügung. Arbeitet der Chirurg bei herausgenommenem Instrument, steht automatisch der an sich für dieses vorgesehene Führungskanal für das Spülen und Absaugen zur Verfügung, d.h. es bedarf hierfür keiner bewußten Manipulationen seitens des Chirurgen.

Sollte der Chirurg bei in den Führungskanal eingeschobenem Instrument während einer Operation feststellen, daß der normale Spül- oder Absaugkanal nicht genügend Kapazität hat oder gar verstopft ist, kann er diesem Mangel durch ein zeitweises Herausnehmen des Instrumentes und das dadurch erfolgende Verbinden des Führungskanals mit dem Spül- bzw. Sauganschluß abhelfen. Die Einsatzmöglichkeiten der erfindungsgemäßen Vorrichtung sind dadurch ohne Querschnittsvergrößerung im Bereich des in das Operationsfeld einzuführenden Schutzrohres wesentlich universeller.

Das Instrument kann auch durch ein durch den Führungskanal hindurchgeführtes Endoskop gebildet sein.

Die Erfindung wird im folgenden beispielsweise anhand der Zeichnung beschrieben; in dieser zeigt:
- Fig. 1: eine schematische perspektivische Ansicht einer erfindungsgemäßen chirurgischen laparoskopischen Vorrichtung, wobei im Handgriff ein Zweistellungs-Dreiwegeventil schematisch im Schnitt wiedergegeben ist,
- Fig. 1a: den durch eine strichpunktierte Linie Ia in Fig. 1 umrandeten Bereich in vergrößerter Darstellung,
- Fig. 2: eine vergrößerte Schnittansicht des in Fig. 1 im Schnitt dargestellten Zweistellungs-Dreiwegeventils bei eingeschobenem Instrument und
- Fig. 3: eine entsprechende Schnittansicht wie Fig. 2 bei herausgezogenem Instrument.

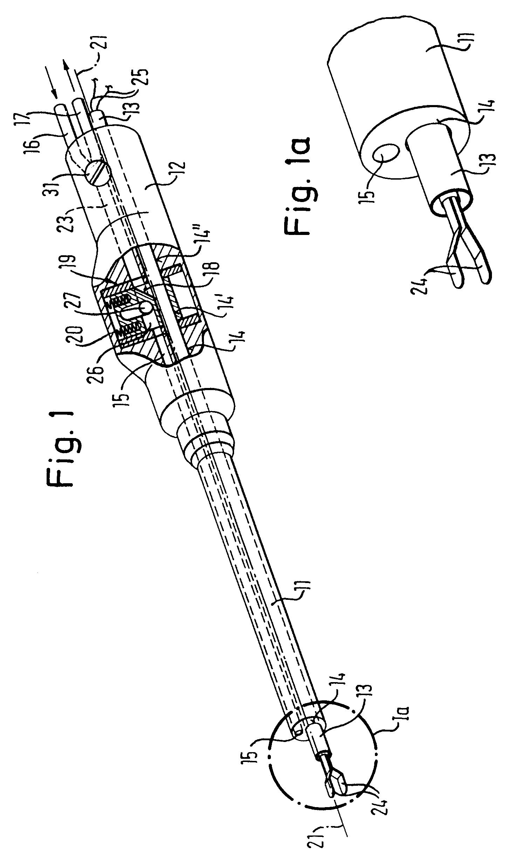

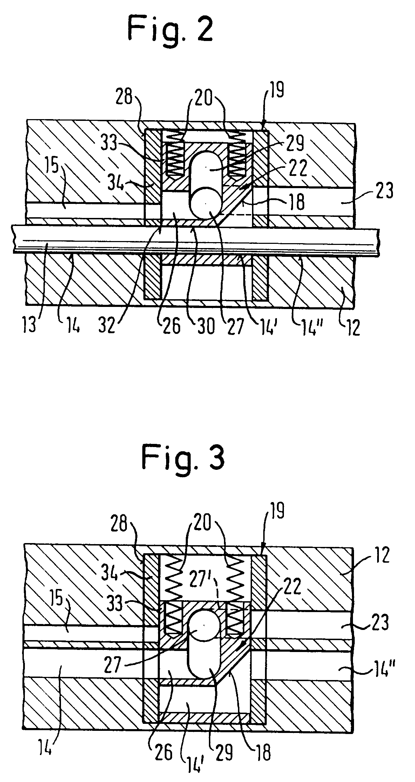

Nach Fig. 1 weist die erfindungsgemäße chirurgische laparoskopische Vorrichtung einen Handgriff 12 auf, an dessen Vorderseite vorzugsweise lösbar ein Schutzrohr 11 befestigt ist, in dem sich nach den Fig. 1, 1a ein zentraler axialer Führungskanal 14 und parallel daneben ein Spül- oder Saugkanal 15 befinden. Die Kanäle 14, 15 setzen sich in der aus Fig. 1 ersichtlichen Weise in den Handgriff 12 fort und münden zunächst an einen in den Handgriff 12 eingebauten Zweistellungs-Dreiwegeventil 19, welches in den Fig. 2 und 3 vergrößert wiedergegeben ist. In der in den Fig. 1 und 2 wiedergegebenen Instrumenten-Aufnahmestellung des Ventils 19 setzt sich der Führungskanal 14 durch eine Bohrung 14' in einem weiter unten beschriebenen Schieber 22 in einen dahinter angeordneten Kanal 14'' gleichen Querschnitts fort. Durch die Kanäle 14, 14', 14'' ist ein stabförmiges Instrument 13 von der Hinterseite des Handgriffs 12 so eingeführt, daß sein mit zwei Koagulationselektroden 24 versehenes distales Ende über das vordere Ende des Schutzrohres 11 vorsteht.

An das hintere Ende des stabförmigen Instrumentes 13 können gemäß Fig. 1 zwei Hochfrequenzspannung führende elektrische Leitungen 25 angelegt sein, über die die Koagulationselektroden 24 mit einem geeigneten Hochfrequenz-Koagulationsstrom versorgt werden.

Das Ventil 19 besteht aus einer in einer Querbohrung 28 des Handgriffs 12 befestigten Buchse 34 und einem gleitend darin untergebrachten Schieber 22.

Im Anschluß an die hintere Mündung des Spül- oder Saugkanals 15 weist Schieber 22 nach den Fig. 1, 2 ein im Querschnitt erweitertes Kanalstück 26 auf, dessen Querschnitt gleich oder größer dem des Führungskanals 14 ist. Das Kanalstück 26 schließt hinten an ein Querlangloch 29 an, dessen Querschnitt ebenfalls mindestens so groß wie der des Führungskanals 14 sein soll und welches sich ebenfalls im Schieber 22 befindet.

Seitlich ausgerichtet mit dem Querlangloch 29 ist eine sich senkrecht zur Richtung des Langloches 29 erstreckende Querbohrung 27 im Handgriff 12 mit gleich großem Querschnitt wie das Kanalstück 26.

Die Querbohrung 27 steht mit einem etwa in Verlängerung des Saug- oder Spülkanals 15 verlaufenden Verbindungskanal 23 innerhalb des Handgriffs 12 in Verbindung, welcher einen Querschnitt aufweist, der mindestens gleich dem des Führungskanals 14 ist.

Der Querschieber 22 ist durch Schraubendruckfedern 20 in Richtung auf das in Fig. 1 neben dem Querschieber 22 angeordnete stabförmige Instrument 13 vorgespannt, so daß der Schieber 22 mit seiner dem Instrument 13 zugewandten Fläche 30 bei eingeschobenem Arbeitswerkzeug 13 gemäß den Fig. 1 und 2 an diesem gleitend anliegt.

Der Verbindungskanal 23 führt zu einem im hinteren Bereich des Handgriffs 12 vorgesehenen Umschaltventil 31 (Fig. 1), welches in nicht dargestellter Weise von außen betätigbar ist und es gestattet, den Verbindungskanal 23 wahlweise mit einem äußeren Spülanschluß 16 oder einem äußeren Sauganschluß 17 zu verbinden. An die Anschlüsse 16, 17 können Schläuche angelegt werden, die entweder mit einer Spülflüssigkeit gespeist oder mit einer Absaugpumpe verbunden sind.

Die Arbeitsweise des beschriebenen Instrumentes ist wie folgt:
In der Arbeitsposition nach den Fig. 1 und 2 ragt die Arbeitsspitze des stabförmigen Instruments 13 aus dem vorderen Ende des Schutzrohres 11 hervor, und mittels der Koagulationselektroden 24 kann eine Operation durchgeführt werden. Durch nicht dargestellte Betätigungsmittel können die Elektroden 24 innerhalb des stabförmigen Instrumentes 13 zurückgezogen werden, wodurch eine zangenartige Bewegung der Elektroden 24 aufeinander zu hervorgerufen wird.

Während oder nach der Operation kann durch den Spülwasseranschluß 16 über die Ventile 31, 19 und den Spülwasserkanal 15 Spülwasser in das Operationsfeld vor der Spitze des Schutzrohres 11 geleitet werden. Durch Umschaltung des Ventils 31 in die Saugstellung kann über den Sauganschluß 17 Flüssigkeit aus dem Operationsfeld durch den Saugkanal 15 und die Ventile 19, 31 abgesaugt werden. In der aus den Fig. 1 und 2 ersichtlichen Arbeitsposition wird der Schieber 22 durch das Instrument 13 in der gegen die Kraft der Federn 20 zurückgeschobenen Lage gehalten, in welcher der Spül- oder Saugkanal 15 mit dem Kanalstück 26 ausgerichtet ist, während der Führungskanal 14 gegenüber dem Kanalstück 26 durch die Dichtkante 32 abgedichtet ist.

Wird aus der Stellung nach Fig. 2 das stabförmige Instrument 13 axial herausgezogen, drücken die Federn 20 den Schieber 22 aus der Position nach Fig. 2 in diejenige nach Fig. 3, wodurch das Kanalstück 26 mit dem Führungskanal 14 in axiale Ausrichtung kommt, während das Ende des Spül- oder Saugkanals 15 durch einen Flansch 33 des Schiebers 22 abgedichtet wird.

Auf diese Weise ist die Strömungsverbindung zwischen dem Saug- oder Spülkanal 15 und der Querbohrung 27 unterbrochen, während nunmehr der vor dem Ventil 19 befindliche Teil des Führungskanals 14 über das gleichen Querschnitt aufweisende Kanalstück 26, das Querlangloch 29, die Querbohrung 27 und den Verbindungskanal 23 mit dem Ventil 31 nach Fig. 1 in Verbindung steht. Durch geeignete Stellung des Ventils 31 kann nun der Führungskanal 14 wahlweise mit dem Spülanschluß 16 oder dem Sauganschluß 17 verbunden werden.

Dies ist insbesondere beim Absaugen von Sekret aus dem Operationsfeld zweckmäßig, weil hierbei häufig größere Stücke mitgerissen werden, die den schmalen Saugkanal 15 verstopfen könnten. Die Verstopfungsgefahr ist bei dem im Querschnitt wesentlich größeren Führungskanal 14 deutlich geringer.

Während der geringe Querschnitt des Spül- oder Saugkanals 15 innerhalb des Schutzrohres 11 durch dessen Funktion bedingt ist, besteht keine Schwierigkeit, die Querschnitte des Kanalstücks 26, der Querbohrung 27, des Querlangloches 29 und des anschließenden Verbindungskanals 23 so groß zu machen, daß hierdurch der vom Führungskanal 14 vorgegebene Strömungsquerschnitt nicht eingeengt wird.

Bei der Ventilposition nach Fig. 3 erstreckt sich eine am hinteren Ende des Querschiebers 22 vorgesehene Schrägfläche 18 quer über die hintere Fortsetzung 14'' des Führungskanals 14. Wird nun vom hinteren Ende des Handgriffs 12 durch die Fortsetzung 14'' hindurch das Instrument 13 eingeschoben, so stößt dessen vorderes Ende an die Schrägfläche 18 und erzeugt dadurch eine Kraft, die den Querschieber 22 gegen die Vorspannkraft der Federanordnung 20 seitlich verschiebt, bis das Kanalstück 26 wieder mit dem Saug- oder Spülkanal 15 und der im Querschieber 22 neben dem Kanalstück 26 vorgesehene Kanalteil 14' wieder mit dem Führungskanal 14 axial ausgerichtet ist. Nunmehr kann das Vorschieben des Instrumentes 13 in den Führungskanal 14 bis in die aus Fig. 1 ersichtliche Position fortgesetzt werden, wo das Instrument 13 durch nicht dargestellte Fixierungsmittel in der gewünschten Axialposition festgelegt werden kann. Nunmehr sind die Spül- bzw. Sauganschlüsse 16, 17 strömungmäßig wieder mit dem Spül- oder Saugkanal 15 verbunden.

In der vorangehenden Beschreibung ist das Umschaltventil 31 als im Handgriff 12 angeordnet beschrieben.

Es besteht jedoch auch die Möglichkeit, das Umschaltventil außerhalb des Handgriffes unterzubringen, z.B. in einem zur von Hand zu haltenden Vorrichtung gehörenden Versorgungsgerät oder in einem separaten Schaltkasten, wobei das Umsteuerventil über eine im Handgriff oder an geeigneter anderer Stelle vorgesehene Taste und elektrische Schaltmittel angesteuert werden kann.

Für den Fall, daß das Umsteuerventil außerhalb des Handgriffes 12 vorgesehen ist, genügt ein einziger Anschluß 16, 17, um wahlweise Spülflüssigkeit zuzuführen oder aus dem Operationsbereich abgesaugte Flüssigkeit abzuführen.

Während in Fig. 1 die Anschlüsse 16, 17 als am hinteren Ende des Handgriffes 12 in dessen Längsrichtung austretend dargestellt sind, ist es bevorzugt, die Anschlüsse 16, 17 auf Ventilhöhe quer aus dem Handgriff 12 herauszuführen.

### Bezugszeichenliste

- 11: Schutzrohr
- 12: Handgriff
- 13: Instrument
- 14: Führungskanal
- 15: Spül- oder Saugkanal
- 16: Spülanschluß
- 17: Sauganschluß
- 18: Schrägfläche
- 19: Zweistellungs-Dreiwegeventil
- 20: Rückstellfeder
- 21: Achse
- 22: Schieber
- 23: Verbindungskanal
- 24: Koagulationselektrode
- 25: elektrische Leitungen
- 26: Kanalstück
- 27: Querbohrung
- 28: Querbohrung
- 29: Querlangloch
- 30: Fläche
- 31: Umschaltventil
- 32: Dichtkante
- 33: Flansch
- 34: Buchse

## Patentansprüche

1. Chirurgische laparoskopische Vorrichtung mit einem Handgriff (12) und einem an dessen Vorderseite angebrachten Schutzrohr (11), wobei in einem im Schutzrohr (11) und dem Handgriff (12) vorgesehenen axialen Führungskanal (14) wenigstens ein stabförmiges Instrument (13) axial herausnehmbar und parallel dazu ein Spül- oder Saugkanal (15) angeordnet sind, welcher durch den Handgriff (12) hindurch zu einem äußeren Spül- bzw. Sauganschluß (16, 17) geführt ist,
dadurch **gekennzeichnet,**
daß im Handgriff (12) neben dem Führungskanal (14) ein Ventil, insbesondere ein Zweistellungs-Dreiwegeventil (19) angeordnet ist, welches in der einen Stellung den Spül- oder Saugkanal (15) mit dem Spül- bzw. Sauganschluß (16 bzw. 17) und in der anderen Stellung den Führungskanal (14) mit dem Spül- oder Sauganschluß (16 bzw. 17) verbindet.

2. Vorrichtung nach Anspruch 1,
dadurch **gekennzeichnet,**
daß selbsttätige Schaltmittel (18, 20) vorgesehen sind, welche bei in den Führungskanal (14) eingeführten Instrument (13) das Ventil (19) in die Stellung verschieben, bei welchem der Spül- oder Saugkanal (15) mit dem Spül- bzw. Sauganschluß (16 bzw. 17) verbunden ist, und bei aus den Führungskanal (14) herausgenommenen Instrument (13) das Ventil (19) in die Stellung verschieben, in der das Ventil (19) den Führungskanal (14) mit dem Spül- bzw. Sauganschluß (16 bzw. 17) verbindet.

3. Vorrichtung nach Anspruch 1 oder 2,
dadurch **gekennzeichnet,**
daß das Ventil (19) auf die Stellung zu, in der es den Führungskanal (14) mit dem Spül- bzw. Sauganschluß (16 bzw. 17) verbindet, vorgespannt ist und Stellmittel (18) vorgesehen sind, welche bei in den Führungskanal (14) eingeschobenem Instrument (13) das Ventil (19) gegen die Vorspannung in die Stellung umschalten, in der das Ventil (19) den Spül- oder Saugkanal (15) mit dem Spül- bzw. Sauganschluß (16 bzw. 17) verbindet.

4. Vorrichtung nach einem der vorhergehenden Ansprüche,
dadurch **gekennzeichnet,**
daß das Ventil (19) einen quer zur Instrumentenachse (21) verlaufenden Schieber (22) aufweist, welcher durch eine Federanordnung (20) auf eine Stellung zu vorgespannt ist, in der der Führungskanal (14) mit einem zum Spül- bzw. Sauganschluß (16 bzw. 17) führenden Verbindungskanal (23) verbunden ist, und in dieser Position in den Führungskanal (14) hineinragt, wobei eine am Schieber (22) vorgesehene, mit dem Führungskanal (14) ausgerichtete Schrägfläche (18) beim Einschieben des stabförmigen Instrumentes (13) eine Verschiebung des Schiebers (22) entgegen der Stellkraft der Federanordnung (20) in die Stellung bewirkt, in der der Schieber (22) den Spül- oder Saugkanal (15) mit dem Verbindungskanal (23) verbindet.

5. Vorrichtung nach einem der vorhergehenden Ansprüche,
dadurch **gekennzeichnet,**
daß zwischen dem Ventil (19) und einem Spül- und einem Sauganschluß (16, 17) oder vor dem Spül- und Sauganschluß (16, 17) ein Umschaltventil (31) zum wahlweisen Verbinden des Spülanschlusses (16) oder des Sauganschlusses (17) mit dem Ventil (19) im Handgriff (12) vorgesehen ist.
